# FASCICULE DE BREVET EUROPEEN

(11) **EP 2 874 636 B1**
(45) Date de publication et mention de la délivrance du brevet: **25.12.2019**
(21) Numéro de dépôt: 13756560.2
(22) Date de dépôt: 09.07.2013
(51) Int. Cl.: A61K 36/064, A61P 15/02, A61P 31/10

(54) **LEVURE SACCHAROMYCES CEREVISIAE POUR PREVENIR ET/OU TRAITER LES MYCOSES VAGINALES**
SACCHAROMYCES CEREVESIAE HEFE FÜR DIE PRÄVENTION UND/ODER BEHANDLUNG VON VAGINALER MYKOSE
SACCHAROMYCES CEREVESIAE YEAST FOR THE PREVENTION AND/OR TREATMENT OF VAGINAL MYCOSIS

(30) Priorité: 13.07.2012 FR 1256799
(43) Date de publication de la demande: 27.05.2015
(73) Titulaire: Lesaffre et Compagnie, 75001 Paris (FR)
(72) Inventeur: VANDEKERCKOVE, Pascal, F-59650 Villeneuve D'ascq (FR)
(74) Mandataire: Plasseraud IP
(86) Numéro de dépôt international: PCT/FR2013/051643
(87) Numéro de publication internationale: WO 2014/009656

(56) Documents cités:
- WO-A2-2009/103884
- BR-A- 0 002 018
- US-A1- 2007 059 298
- PAPAEMMANOUIL V ET AL: "Prevalence and susceptibility ofcausing vaginitis in Greek women", ANAEROBE, LONDON, GB, vol. 17, no. 6, 13 avril 2011 (2011-04-13) , pages 298-299, XP028351585, ISSN: 1075-9964, DOI: 10.1016/J.ANAEROBE.2011.04.008 [extrait le 2011-04-29]

## Description

La présente invention concerne une nouvelle utilisation thérapeutique d'une levure *Saccharomyces cerevisiae,* à savoir une utilisation pour la prévention et/ou le traitement des mycoses vaginales.

Les mycoses vaginales ou vulvo-vaginales sont provoquées principalement par une infection par des champignons pathogènes, le plus souvent *Candida albicans,* au niveau de la zone intime des femmes.

Les mycoses vaginales sont considérées à ce jour comme un important problème de santé public et constituent la deuxième cause d'infection vaginale après les vaginites bactériennes.

Il est estimé que 75% des femmes développent un jour ou l'autre une mycose vaginale. Environ 50% d'entre elles subissent au moins 2 à 3 épisodes de mycoses vaginales au cours de leur vie. 10 à 20% des femmes souffrent de mycoses vaginales récidivantes avec en moyenne 4 épisodes par an.

En l'an 2000 il a été estimé aux Etats-Unis que les coûts médico-économiques du traitement de cette pathologie étaient de l'ordre d'un milliard de dollars par an.

Les traitements antimycotiques habituellement utilisés à ce jour sont basés sur l'emploi de molécules thérapeutiques, principalement des dérivés azoles tels que par exemple fluconazole, kétoconazole, éconazole, miconazole ou clotrimazole, administrés généralement sous forme de crème ou d'ovule à introduire dans le vagin. Dans certains cas, un traitement par voie orale vient compléter le traitement par voie locale.

Cependant, un des problèmes majeurs de ce type de traitement est que, lors de mycoses vaginales à répétition, le champignon pathogène, en particulier *Candida albicans,* peut développer des résistances aux traitements antimycotiques existants.

Un autre problème dans l'utilisation de dérivés azoles pour soigner les mycoses est la sensation d'irritation et de cuisson au niveau de la vulve.

Des publications récentes ont montré que l'utilisation de probiotiques *(Lactobacillus rhamnosus* GR-1 & *Lactobacillus reuteri* RC14) associés à la molécule fluconazole permettait d'augmenter l'efficacité du fluconazole dans le traitement des mycoses (Martinez et al., Letters in Applied Microbiology, 2009, (48) 269-274) .

Il est rappelé que l'effet probiotique d'une souche donnée, qu'il s'agisse d'une souche de bactérie ou d'une souche de levure, est spécifique de cette souche, et non du genre et de l'espèce considéré.

Par ailleurs, il est connu que si majoritairement les mycoses vaginales sont causées par *Candida albicans,* des cas d'infections causées par *Saccharomyces cerevisiae* ont été décrits (Savini et al., Mycopathologia, 2008, 166 :47-50).

Le document BR 0 002 018 A divulgue l'utilisation de *Saccharomyces cerevisiae* pour le traitement des infections vaginales causées par des micro-organismes pathogènes tels que des protozoaires, champignons et/ou bactérie. Plus particulièrement, la souche utilisée est une souche *Saccharomyces cerevisiae* variété *boulardii.*

Les objets de l'invention sont définis dans les revendications. Tous les autres objets sont décrits dans un but illustratif, et les indications que ces objets constitueraient l'invention, ou toute indication similaire, doivent être perçues dans le contexte de la demande telle que déposée, ne changeant pas l'étendue de protection des revendications.

L'un des objectifs de la présente invention est donc de proposer de nouveaux médicaments ou compléments nutritionnels à effet santé destinés à la prévention et/ou au traitement de la mycose vaginale.

Un autre objectif de l'invention est de proposer de nouveaux médicaments ou compléments nutritionnels à effet santé destinés au traitement de la mycose qui ne présentent pas les inconvénients susmentionnés liés à la prise de dérivés azoles (phénomène de résistance, effets secondaires tels qu'irritation, brûlure, ...), tout en présentant une efficacité au moins comparable aux traitements existants.

Les présents inventeurs ont trouvé de manière surprenante que l'utilisation de la souche *Saccharomyces cerevisiae* déposée le 17 octobre 2007 auprès de la CNCM (Collection Nationale de Cultures de Microorganismes, 25, rue du Docteur Roux, 75724 Paris cedex 15, France) sous le numéro I-3856, permettait d'atteindre ces objectifs.

Ainsi, l'invention porte sur la souche *Saccharomyces cerevisiae* déposée par la Société demanderesse auprès de la CNCM sous le numéro I-3856 pour son utilisation dans la prévention et/ou le traitement des mycoses vaginales, et plus particulièrement des candidoses vaginales.

La souche *Saccharomyces cerevisiae* déposée sous le numéro I-3856 a précédemment été décrite par la Demanderesse dans le document WO 2009/103884. Cette souche a plus particulièrement été décrite pour son utilisation dans la prévention et/ou le traitement de pathologies, désordres ou troubles de l'intestin.

Cependant, le comportement des champignons pathogènes et des probiotiques vis-à-vis de la muqueuse intestinale n'est pas transposable à celui adopté vis-à-vis de la muqueuse vaginale, l'environnement de ces muqueuses notamment étant différent.

A la connaissance de la Demanderesse, c'est la première fois qu'une souche de *Saccharomyces cerevisiae* est décrite comme ayant un effet dans la prévention et/ou le traitement de mycoses vaginales.

L'expression « souche de levure » désigne une population relativement homogène de cellules de levure.

Une souche de levure est obtenue à partir de l'isolement d'un clone, un clone étant une population de cellules obtenue à partir d'une seule cellule de levure.

Selon un autre mode de réalisation, la présente invention porte sur la levure *Saccharomyces cerevisiae* obtenue par culture de la souche *Saccharomyces cerevisiae* déposée auprès de la CNCM sous le numéro I-3856, pour son utilisation dans la prévention et/ou le traitement des mycoses vaginales, et plus particulièrement des candidoses vaginales.

La levure utilisée selon l'invention est obtenue par culture de la souche numéro I-3856 dans un milieu de culture, par exemple comme décrit dans le livre de référence « Yeast Technology », 2ème édition, 1991, G. Reed et T.W. Nagodawithana, publié par Van Nostrand Reinhold, ISBN 0-442-31892-8.

La levure utilisée selon l'invention peut être obtenue par un procédé à l'échelle industrielle qui comprend les deux étapes suivantes :
- culture d'une souche de levure dans un milieu de culture en plusieurs stades, d'abord en semi-anaérobiose, puis en aérobiose,
- séparation par centrifugation de la levure ainsi produite de son milieu de culture, pour obtenir une crème de levure liquide contenant entre 12 et 25% de matière sèche, voire une quantité plus élevée de matière sèche, en particulier si la crème de levure est mélangée avec des produits osmolytes.

Dans la présente invention, on utilisera indifféremment les termes « levure obtenue par culture de la souche *Saccharomyces cerevisiae* déposée à la CNCM sous le numéro I-3856", "levure obtenue par culture de la souche I-3856" et "levure Sc I-3856", ces trois termes ayant la même signification telle que donnée ci-dessus.

Selon encore un autre mode de réalisation, l'invention porte sur un dérivé de la levure obtenue par culture de la souche *Saccharomyces cerevisiae* déposée auprès de la CNCM sous le numéro I-3856, pour son utilisation dans la prévention et/ou le traitement des mycoses vaginales, en particulier des candidoses vaginales, ledit dérivé étant choisi parmi la paroi de ladite levure, les glucanes pariétaux de ladite levure, les mannoprotéines pariétales de ladite levure ou leurs mélanges.

La paroi de levure désigne de manière large à la fois la paroi et la membrane plasmique de la levure.

De façon classique, la paroi de levure est obtenue par un procédé comprenant une étape d'autolyse de la levure suivie d'une étape de séparation de la fraction soluble de la fraction insoluble, la fraction insoluble isolée correspondant à la paroi de levure qui peut ensuite être séchée.

La présente description a particulièrement pour objet une paroi de levure Sc I-3856, pour son utilisation dans la prévention et/ou le traitement de mycoses vaginales, à une dose quotidienne de 1 mg à 10 g, et de préférence de 100 mg à 1 g.

La présente description a également pour objet des glucanes pariétaux de levure Sc I-3856, pour leur utilisation dans la prévention et/ou le traitement de mycoses vaginales, à une dose quotidienne de 0,25 mg à 2,5 g, et de préférence de 25 mg à 250 mg.

La présente description a particulièrement pour objet des mannoprotéines pariétales de levure Sc I-3856, pour leur utilisation dans la prévention et/ou le traitement de mycoses vaginales, à une dose quotidienne de 0,1 mg à 1 g, et de préférence de 10 mg à 100 mg.

On entend par « mycose vaginale », toute infection au niveau de la zone vaginale provoquée par des champignons.

Le terme « vaginal(e) » est utilisé de manière large et les termes « vaginal(e) » et « vulvo-vaginal(e) » sont ici synonymes.

On entend par "candidose vaginale" un type particulier de mycose vaginale due à des champignons du genre *Candida,* comme par exemple ceux choisis dans le groupe comprenant *Candida albicans, Candida tropicalis, Candida pseudotropicalis, Candida krusei, Candida glabrata* et leurs mélanges.

La candidose vaginale est également appelée vaginite candidosique.

La présente invention a ainsi également pour objet une souche telle que définie ci-dessus, une levure telle que définie ci-dessus ou un dérivé tel que défini ci-dessus, pour son utilisation dans la prévention et/ou le traitement des candidoses vaginales, comme par exemple les candidoses dues à des champignons choisis dans le groupe comprenant *Candida albicans, Candida tropicalis, Candida pseudotropicalis, Candida krusei, Candida glabrata* et leurs mélanges.

La levure Sc I-3856 peut être utilisée de manière préventive chez des femmes présentant des prédispositions et/ou une sensibilité aux mycoses vaginales, notamment aux candidoses vaginales, ou de manière curative, par exemple lors d'épisodes infectieux.

Sans vouloir être liés par aucune théorie, les présents inventeurs sont d'avis que l'administration d'une levure obtenue par culture de la souche I-3856 permet d'inhiber la multiplication des *Candida* au niveau vaginal, notamment en diminuant l'adhérence de *Candida* à la muqueuse vaginale. De plus, les structures polysaccharidiques pariétales, en particulier les glucanes et les mannanes, joueraient un rôle dans ce mode d'action.

Selon un mode de réalisation avantageux de l'invention, la levure Sc I-3856 utilisée est vivante.

La levure vivante se présente alors par exemple sous forme d'une levure fraîche ou sous forme d'une levure sèche.

La présente description a notamment pour objet la levure Sc I-3856 sous forme de levure sèche ou de levure fraîche, pour son utilisation dans la prévention et/ou le traitement de mycoses vaginales, par exemple à une dose quotidienne de 1.10⁷ à 1.10¹¹ UFC, et de préférence de 1.10⁹ à 1.10¹⁰ UFC.

Une levure fraîche est caractérisée par une teneur élevée en eau.

Une levure fraîche est par exemple choisie parmi une levure pressée et une levure liquide.

Une levure liquide selon l'invention, appelée également « crème de levure liquide » est une suspension aqueuse comprenant de 12% à 50% de matière sèche levure, plus généralement de 12% à 22% de matière sèche levure.

Une levure pressée selon l'invention est obtenue par :
- filtration d'une crème de levure liquide, en général sur un filtre rotatif sous vide, pour obtenir une levure fraîche déshydratée contenant de 26% à 37% de matière sèche,
- malaxage de ladite levure fraîche déshydratée et extrusion.

Une levure pressée comprend de 26% à 37% de matière sèche levure.

La levure pressée peut être émiettée ou non.

Parmi les levures sèches, on peut par exemple citer la levure sèche active, la levure sèche instantanée, et la levure à humidité intermédiaire surgelée.

Un avantage d'une levure sèche est sa longue durée de conservation.

Une levure sèche active ou une levure sèche active instantanée selon l'invention comprend un taux de matière sèche levure supérieur à 90%, de préférence un taux de matière sèche compris de 92% à 96%.

La levure sèche active est obtenue par déshydratation de la levure pressée ou liquide par l'action conjointe de la chaleur (à basse température) et d'une activité mécanique, qui permettent de transformer un produit pâteux (levure pressée ou liquide) en un produit sec, sous forme de sphérules.

Par exemple, la levure sèche active est obtenue par extrusion et séchage en lit fluidisé d'une levure pressée ou d'une levure liquide.

La levure sèche active se présente sous forme de sphérules dont le diamètre est généralement compris de 0,1 µm à 2,5 mm.

La levure sèche instantanée est obtenue par déshydratation de la levure pressée ou liquide par l'action d'un gradient d'air chaud qui permet de transformer un produit pâteux (levure pressée ou liquide) en de fins vermicelles secs. La levure sèche instantanée doit ensuite, pour rester stable, être conditionnée en absence d'oxygène.

La levure sèche à humidité intermédiaire surgelée comprend par exemple de 70% à 85% de matière sèche levure.

Dans un mode de réalisation préféré, la levure Sc I-3856 utilisée selon l'invention dans la prévention et/ou le traitement des mycoses vaginales, se présente sous forme de levure sèche active ou sous forme de levure sèche instantanée.

Selon un autre mode de réalisation avantageux de l'invention, la levure Sc I-3856 utilisée selon l'invention dans la prévention et/ou le traitement des mycoses vaginales se présente sous forme de levure morte, appelée également levure désactivée.

Une levure morte est une levure dont le métabolisme est irrémédiablement arrêté.

Une levure morte peut être obtenue par des techniques bien connues de l'homme du métier, telles qu'un traitement thermique de la levure, un traitement consistant à soumettre la levure à plusieurs cycles de congélation et décongélation successives, un traitement par irradiation, un traitement par atomisation ou une combinaison de ces traitements.

La présente description a également pour objet la levure Sc I-3856 sous forme de levure morte, pour son utilisation dans la prévention et/ou le traitement de mycoses vaginales, à une dose quotidienne de 1 mg à 10 g, et de préférence de 100 mg à 1 g.

La présente description a également pour objet une souche telle que définie ci-dessus, une levure telle que définie ci-dessus ou un dérivé tel que défini ci-dessus, pour son utilisation dans la prévention et/ou le traitement de mycoses vaginales, ladite souche, ladite levure ou ledit dérivé étant administré par voie orale ou par voie vaginale, de préférence par voie vaginale.

L'invention porte également sur une composition comprenant la levure Sc I-3856 et/ou un dérivé de ladite levure, pour une utilisation dans la prévention et/ou le traitement de mycoses vaginales.

La composition selon l'invention peut être une composition pharmaceutique, un complément nutritionnel à effet santé ou une composition alimentaire.

L'invention porte également sur une composition comprenant la levure Sc I-3856 et/ou un dérivé de ladite levure, et éventuellement au moins un excipient pharmaceutiquement acceptable pour une utilisation dans la prévention et/ou le traitement de mycoses vaginales.

Les mycoses vaginales sont telles que définies ci-dessus.

La composition utilisée selon l'invention est destinée à une administration par voie orale ou vaginale, la voie vaginale étant préférée.

A titre d'exemple de composition se présentant sous une forme adaptée pour la voie orale on peut citer un comprimé, une capsule, une gélule, un sachet, une poudre, une crème, un sirop ou une ampoule.

A titre d'exemple de composition se présentant forme adaptée pour la voie vaginale on peut citer un ovule, une capsule, une gélule, une crème ou un comprimé.

Les excipients mis en œuvre dans la composition selon l'invention sont des excipients classiquement utilisés qui sont adaptés à la préparation de formes orales ou vaginales.

Le dosage journalier dépend à la fois du type de mycoses, du mode d'administration (voie orale ou voie vaginale) et du type de traitement curatif ou préventif.

Selon un mode de réalisation particulier de l'invention, la composition comprend la levure Sc I-3856 sous forme de levure morte, pour une utilisation quotidienne dans une quantité de 1 mg à 10 g, et de préférence de 100 mg à 1 g.

Selon un autre mode de réalisation de l'invention, la composition comprend la levure Sc I-3856 sous forme sèche ou fraîche, pour une utilisation quotidienne dans une quantité de 1.10⁷ à 1.10¹¹ UFC, et de préférence de 1.10⁹ à 1.10¹⁰ UFC.

Selon un autre mode de réalisation, la composition comprend une paroi de levure Sc I-3856, pour une utilisation quotidienne dans une quantité de 1 mg à 10 g, et de préférence de 100 mg à 1 g.

Selon un autre mode de réalisation, la composition comprend des glucanes pariétaux de levure Sc I-3856, pour une utilisation quotidienne dans une quantité de 0,25 mg à 2,5 g, et de préférence de 25 mg à 250 mg.

Selon un autre mode de réalisation, la composition comprend des mannoprotéines pariétales de levure Sc I-3856, pour une utilisation quotidienne dans une quantité de 0,1 mg à 1 g, et de préférence de 10 mg à 100 mg.

La dose efficace journalière peut être administrée en une, deux ou trois fois.

La présente invention a encore pour objet une composition pharmaceutique comprenant la levure Sc I-3856 et/ou le dérivé de la levure Sc I-3856 en association avec un autre principe actif et éventuellement un excipient pharmaceutiquement acceptable, pour une utilisation dans la prévention et/ou le traitement des mycoses vaginales.

Le principe actif utilisé en association avec la levure Sc I-3856 peut être un principe actif ayant une activité apaisante, anti-irritante, analgésique, antalgique, anti-inflammatoire, cicatrisante, antifongique, antimycosique et/ou antimycotique.

Lorsque la composition pharmaceutique comprend la levure Sc I-3856 et/ou le dérivé de la levure Sc I-3856 en association avec un principe actif ayant une activité antifongique, ledit principe actif ayant une activité antifongique n'a pas d'effet sur *Saccharomyces cerevisiae.*

Les termes « antifongique », « antimycosique » et « antimycotique » sont ici synonymes.

La présente invention va maintenant être illustrée à l'aide des exemples et des figures qui suivent, qui sont donnés à titre d'illustration, ne sont nullement limitatifs et dans lesquels :

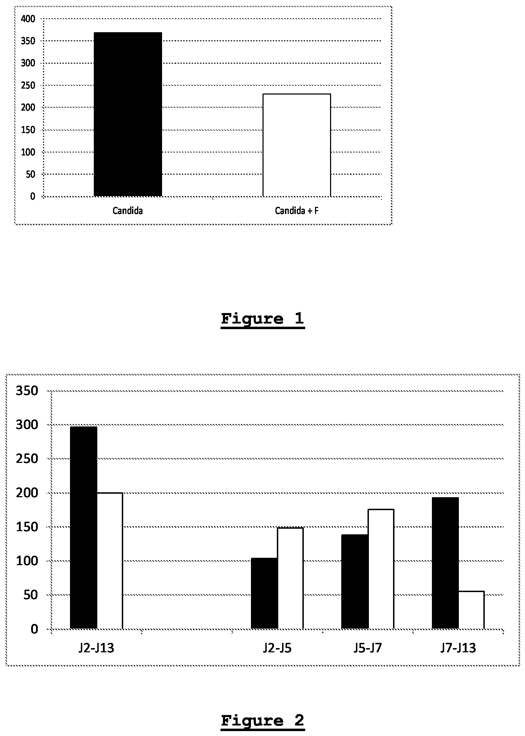

- la figure 1 est un histogramme représentant les valeurs ASC (Aire Sous Courbe) représentatives du nombre de cellules viables de *Candida albicans* trouvées au niveau de la muqueuse vaginale des rats traités au fluconazole (en blanc) ou non traités au fluconazole (en noir) dans les treize premiers jours suivant l'infection par *Candida albicans,*
- la figure 2 est un histogramme représentant les valeurs ASC (Aire Sous Courbe) représentatives du nombre de cellules viables de *Candida albicans* trouvées au niveau de la muqueuse vaginale des rats traités au fluconazole (en blanc) ou non traités au fluconazole (en noir) entre les jours J2-J13, J2-J5, J5-J7 et J7-J13 suivant l'infection par *Candida albicans,*

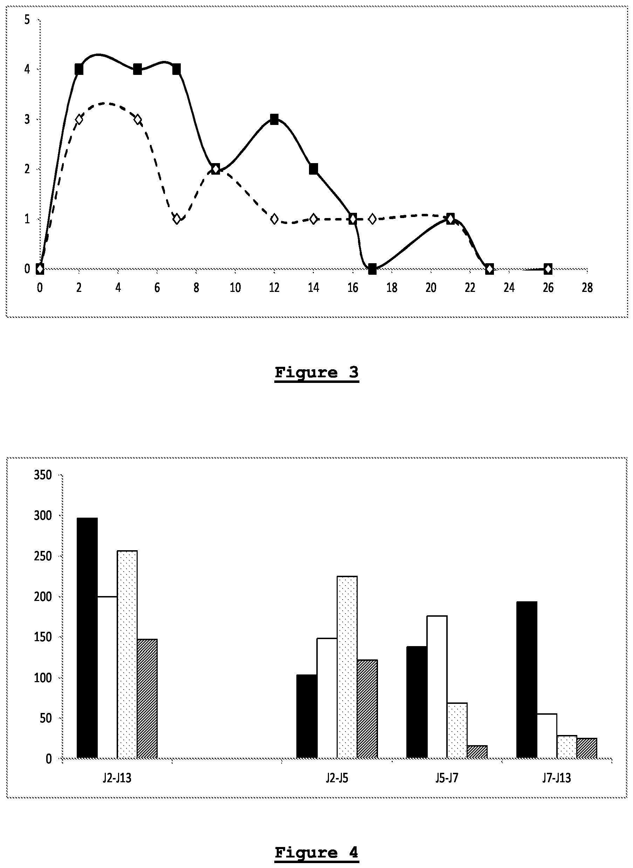

- la figure 3 représente une évaluation semi-quantitative des antigènes de structure mannosidique caractéristique de *Candida albicans* en fonction du temps (en jours), présents chez les rats traités avec du fluconazole (losanges blancs) ou non traités (carrés noirs) après une infection avec *Candida albicans,*
- la figure 4 est un histogramme représentant les valeurs ASC pour les rats non traités (en noir), les rats traités avec du fluconazole (en blanc) ou les rats traités avec la levure Sc I-3856 en traitement préventif (tirets) ou curatif (hachuré), entre les jours J2-J13, J2-J5, J5-J7 et J7-J13 suivant l'infection par *Candida albicans,*

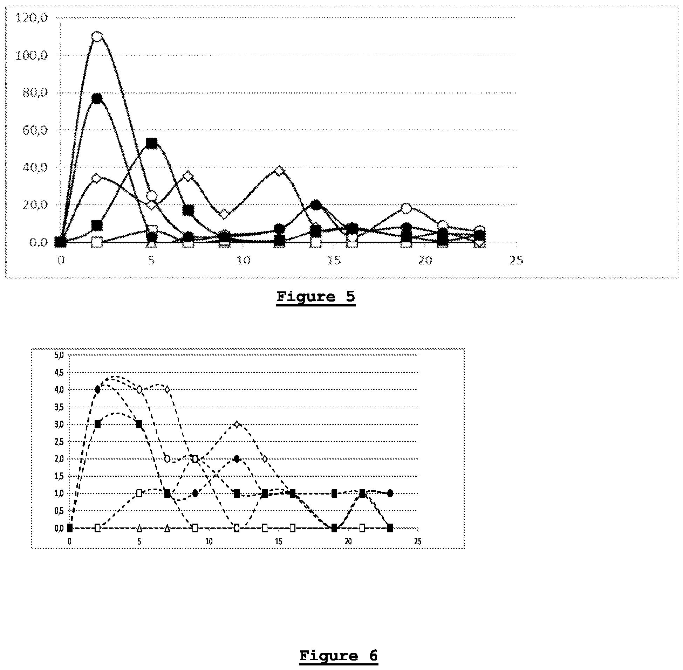

- la figure 5 représente le nombre de cellules viables de *Candida albicans* (en UFC) trouvées dans les liquides de lavement des rats non traités (losanges blancs) ou traités avec du fluconazole (carrés noirs), ou avec la levure Sc I-3856 en traitement préventif (cercles blancs) ou curatif (cercles noirs), après une infection avec *Candida albicans,* et chez les rats contrôle non infectés (triangles blancs) et les rats contrôle non infectés traités avec la levure Sc I-3856 (carrés blancs).
- la figure 6 représente une évaluation semi-quantitative des antigènes de structure mannosidique caractéristique de *Candida albicans* en fonction du temps (en jours), présents respectivement chez les rats non traités (losanges blancs) ou traités avec du fluconazole (carrés noirs), ou avec la levure Sc I-3856 en traitement préventif (cercles blancs) ou curatif (cercles noirs) après une infection avec *Candida albicans,* chez les rats contrôle non infectés (triangles blancs) et les rats contrôle non infectés traités avec la levure Sc I-3856 (carrés blancs).

### EXEMPLE : DEFINITION D'UN MODELE D'INFECTION VAGINALE INDUITE PAR CANDIDA ALBICANS CHEZ DES RATS FEMELLES ET MISE EN EVIDENCE DE L'EFFET DE LA LEVURE Sc I-3856 DANS CE MODELE

Le modèle développé ci-après a permis de démontrer que l'administration de la levure Sc I-3856 chez des rats femelles permet de prévenir et/ou de traiter des mycoses vaginales provoquées par une infection induite par *Candida albicans (C. albicans).*

### MATERIELS ET METHODES

Les expériences sur les animaux sont effectuées dans des établissements agréés de l'Institut Pasteur de Lille, conformément aux directives européennes sur l'expérimentation animale.

Le dénombrement des cellules est effectué selon la méthode du comptage par colonie. Les valeurs sont exprimées en Unité Formant Colonie (UFC).

Les animaux sont logés à 3 par cage avec un libre accès à des aliments standards pour rats ainsi qu'à l'eau du robinet.

Des rats femelles ovariectomisées (Sprague-Dawley) âgées de 3 à 5 semaines sont achetées auprès de Janvier Laboratoires, France. Elles sont maintenues sous pseudo-œstrus par une injection sous-cutanée de benzoate d'œstradiol (Sigma, benzoate d'œstradiol remis en suspension dans huile de sésame ; 0,5 mg / animal) tous les deux jours (soit trois fois par semaine) jusqu'à la fin de l'expérience.

Une semaine après la première injection d'œstradiol induisant des pseudo-œstrus, les rats femelles sont infectés par C. *albicans.* Une infection par C. *albicans* correspond à une administration unique intravaginale de la souche C. *albicans* à la concentration de 10⁷ UFC.

Les rats femelles sont répartis en 6 groupes différents comme indiqué ci-dessous.

| Groupe testé | Infection unique par *C*. *albicans* | Levure Sc I-3856 | Volume | Voie d'application | Nombre d'animaux par groupe |
|---|---|---|---|---|---|
| Véhicule | 0 | 0 | 100 µl | administration intravaginale | 7 |
| *C. albicans* | 10⁷ UFC | 0 | 100 µl | administration intravaginale | 7 |
| *Levure Sc I-3856* | 0 | 10⁷ UFC | 100 µl | administration intravaginale | 7 |
| *C. albicans* + levure Sc I-3856 Préventif | 10⁷ UFC | 10⁷ UFC | 100 µl | administration intravaginale | 7 |
| *C. albicans* + levure Sc I-3856 Curatif | 10⁷ UFC | 10⁷ UFC | 100 µl | administration intravaginale | 7 |
| *Candida* + Fluconazole (50 µg) | 10⁷ UFC | 0 | 100 µl | administration intravaginale | 7 |

La levure Sc I-3856 testée est sous forme d'une levure sèche active.

Le fluconazole est l'agent antifongique de référence, utilisé comme contrôle positif car habituellement utilisé pour traiter la candidose vaginale.

Le fluconazole est administré par voie intra-vaginale à une dose de 50 µg par rat, respectivement 1 h, 24 h et 48 h après l'infection par *C. albicans.*

### Traitement préventif avec la levure Sc I-3856

La levure Sc I-3856 mise en suspension dans un tampon PBS est administrée à la dose de 10⁷ UFC par rat et par administration intravaginale, respectivement 48h, 24h et 1h avant l'infection à *C*. *albicans.*

La levure Sc I-3856 est ensuite administrée tous les 8 jours jusqu'à la fin de l'expérience.

### Traitement curatif avec la levure Sc I-3856

La levure Sc I-3856 mise en suspension dans un tampon PBS est administrée à la dose de 10⁷ UFC par rat et par administration intravaginale, respectivement 1h, 24h et 48h après l'infection à *C. albicans.*

La levure ScI-3856 est ensuite administrée tous les 8 jours jusqu'à la fin de l'expérience.

### Evaluation de la croissance de la colonie de C. albicans

Le nombre de colonies de *C. albicans* adhérente à la muqueuse vaginale est évalué tous les deux jours après l'infection à *Candida albicans* et jusqu'à la fin de l'expérience (3 semaines plus tard) en effectuant un frottis cervico-utérin (avec une öse calibrée) et par étalement sur des plaques Chrom-Agar (un milieu de croissance spécifique et sélectif pour *Candida*), 5 prélèvements vaginaux étant mis en commun.

Les colonies viables de *Candida albicans* sont comptées après deux jours de culture à 30 °C sur un milieu sélectif (plaques Chrom-Agar, BD).

### Evaluation cytologique de l'inflammation

Les frottis sont effectués à partir d'un échantillon cervical et de lavements.

Des préparations de cellules en couche mince sur lames sont préparées à partir de lavements mis en commun dans chaque groupe de rats (lavements de 5 rats) au moyen d'une cytocentrifugeuse (Cytospin®). Les lames sont fixées en utilisant un fixateur aérosol cytologique (Cytoral®).

Un échantillon vaginal est prélevé à partir de 2 rats par groupe 10 jours après l'infection par *C. albicans.*

Une coloration classique May Grunwaid Giemsa est réalisée pour évaluer le niveau de l'inflammation dans les groupes de rats ayant reçu *C. albicans* seul ou associé au traitement avec la levure Sc I-3856.

Des études cytologiques sont également menées et une coloration de Papanicolaou est réalisée afin d'évaluer la colpocytologie hormonale. L'étude est réalisée sur un frottis cervico-utérin.

Une coloration immunohistochimique est également effectuée sur des sections vaginales et frottis cervico-utérin en utilisant des anticorps dirigés contre *C. albicans* (5B2) ou la lectine de *Galanthus nivalis* (GNL) pour la levure Sc I-3856.

### Analyses biologiques

Le pH des lavements vaginaux mis en commun est évalué tous les deux jours à l'aide d'une bande de pH.

La détection d'un antigène de *C. albicans* est utile pour diagnostiquer une candidose vaginale. L'antigène comprend une structure mannosidique fortement immunogène présente dans la paroi cellulaire de *C. albicans.* L'antigène à structure mannosidique est le principal antigène présent lors de l'infection vaginale, il constitue donc un biomarqueur utile pour le diagnostic de la candidose invasive.

Une première possibilité pour détecter la présence de l'antigène à structure mannosidique consiste à utiliser la méthode immuno-enzymatique ELISA (Platelia *Candida* Ag test, Bio-Rad).

Le niveau d'antigène de *C. albicans* est évalué dans les lavements vaginaux mis en commun, dilués au 1/10 avec un traitement standard par la chaleur et selon les instructions des fabricants. La quantité d'antigènes détectée est exprimée en picogrammes par ml.

Une autre possibilité pour détecter la présence de l'antigène à structure mannosidique consiste à utiliser des bandes ICT (Candi Vagi, SR2B). « ICT » est basée sur la capture de l'antigène contenu dans les sécrétions vaginales par l'utilisation d'une immunoglobine de souris IgM dirigée contre l'antigène à structure mannosidique. L'anticorps monoclonal reconnaît les épitopes de l'antigène à structure mannosidique pour un large éventail d'espèces de Candida. L'anticorps monoclonal est conjugué à des particules ou dans une phase mobile et est également appliqué à une bande de nitrocellulose comme anticorps de capture. Le bâton ICT est placé dans la suspension de prélèvement vaginal et les échantillons se déplacent par action capillaire à travers le bloc contenant le conjugué « MAb-or » (Mab pour anticorps monoclonal). L'analyse est effectuée comme décrit dans la publication Marot-Leblond et al., Journal of Clinical microbiologie, 2009.

Le niveau d'inflammation est évalué en mesurant le niveau de calprotectine (une enzyme libérée par les neutrophiles) dans les prélèvements vaginaux et est quantifié à l'aide d'un kit validé pour quantifier la calprotectine humaine dans les selles (kit Labodia).

Les analyses statistiques sont effectuées en utilisant le Test de Permutation pour deux échantillons indépendants. Les statistiques sont calculées à l'aide du logiciel StatXact (Cytel lnc, Cambridge, MA, USA). Les différences sont considérées comme statistiquement significatives si la valeur p est inférieure à 0,05.

### RESULTATS ET DISCUSSION

### AVEC LE CONTROLE FLUCONAZOLE

### Frottis cervico-utérins correspondant à la présence de C. albicans viables dans la muqueuse vaginale des rats.

Le tableau 1 ci-dessous représente la moyenne de la croissance des colonies de *C. albicans* sur des plaques Chrom-Agar dans chaque groupe de rats (5 rats par groupe).

| Durée en jour | 2 | 5 | 7 | 10 | 13 | 15 | 17 | 20 | 23 | 25 |
|---|---|---|---|---|---|---|---|---|---|---|
| Contrôle | 0±0 | 0±0 | 0±0 | 0±0 | 0±0 | 0±0 | 0±0 | 0±0 | 0±0 | 0±0 |
| *C*. *albicans* | 14,75±5, 12 | 16,25±1,43 | 35±9,5 | 49,71±13,51 | 48,4±15,53 | 20,8±9,55 | 26±6,98 | 16±5,37 | 15,6±11,8 | 10,6±4,01 |
| *C*. *albicans*+fluconazole | 5,25±1,37 | 25±6,5 | 42±5,18 | 17±5,29 | 7±2,5 | 34,45±5,26 | 24,4±9,7 | 8,8±4,33 | 11,8±7,55 | 11,6±4,97 |

On peut donc conclure que :
- l'administration de *C. albicans* mène à une infection progressive de la muqueuse vaginale débutant au jour 2 avec une intensité maximale au jour 12 suivie par une phase d'élimination spontanée ; le jour 12 est considéré comme le temps optimal pour l'infection de *C. albicans* dans ce modèle ;
- l'administration de fluconazole mène à une absence d'infection à *C. albicans* au jour 12 avec la présence d'un petit nombre de *C. albicans* dans la muqueuse vaginale observée au jour 7.

Une autre façon d'exprimer les résultats est de calculer l'aire au-dessous de la courbe (ASC) entre le jour 2 et le jour 12 qui représente un marqueur quantitatif de la présence de *C. albicans* viable dans la muqueuse vaginale de rats recevant ou pas le fluconazole.

Le traitement avec le fluconazole diminue de 36% le nombre de *C. albicans* viables dans les 12 premiers jours après l'infection.

Ainsi *C. albicans* mène à une infection vaginale progressive avec un pic d'intensité au jour 12. Le traitement antimycosique avec le fongicide fluconazole conduit à une baisse de 36% de l'infection de la muqueuse vaginale et une prévention de la candidose vaginale au jour 12. Ces résultats sont présentés sur la figure 1.

### Lavements liquides correspondant à l'élimination de C. albicans

Le tableau 2 ci-dessous représente le nombre de *C. albicans* UFCs déterminé à partir des lavements.

| | | | | | | | | | | |
|---|---|---|---|---|---|---|---|---|---|---|
| Durée en jour | 2 | 5 | 7 | 10 | 13 | 15 | 17 | 20 | 23 | 25 |
| Contrôle | 0 | 0 | 0 | 0 | 0 | 0 | 0 | 0 | 0 | 0 |
| *C*. *albicans* | 34 | 20 | 35 | 15 | 38 | 8 | 8 | 3 | 5 | 0 |
| *C*. *albicans* + fluco | 9 | 53 | 17 | 3 | 1 | 6 | 7 | 3 | 1 | 4 |

On peut donc conclure que les *C. albicans* viables (UFC) sont régulièrement et constamment libérés dans les lavements vaginaux entre le jour 2 et 16 (environ 30 UFC).

Le traitement avec le fluconazole augmente la libération de *Candida* au cours des 8 premiers jours après l'infection avec un pic d'intensité au jour 5.

Une autre façon d'exprimer les résultats est de calculer l'aire sous la courbe (ASC) entre le jour 2 et le jour 12 qui est un marqueur quantitatif de l'élimination des *C. albicans* viables dans le lavement vaginal de rats recevant ou pas le fluconazole.

Autrement dit, la valeur ASC est une valeur représentative du nombre viable de *C. albicans* trouvés dans la muqueuse vaginale des rats.

L'activité anti-mycostatique du fluconazole réduit de 20% l'élimination des *C. albicans* viables entre les jours 2 à 12 chez les rats traités par rapport aux rats non traités (voir figure 2).

Le traitement au fluconazole augmente l'élimination des *C. albicans* viables de 45% dans la première semaine après l'infection.

Si l'expérience est décomposée en différentes étapes, on observe que le fluconazole induit dans un premier temps une libération rapide et élevé de *C. albicans* dans le lavement (Jours 2-5 et 5-7) par rapport aux rats non traités (voir figure 2).

En fait, le traitement avec le fluconazole induit une libération de *C. albicans* deux fois plus importante (73%) que la libération naturelle (34,6%) observée chez les rats infectées mais non traités (34,6%) entre les jours 2 à 5 indiquant que l'effet de fluconazole est un effet premier ou précoce.

L'antigène à structure mannosisique est quantifié à l'aide des bandes non commerciales dans des lavements mis en commun de 5 rats.

La quantité d'antigènes à structure mannosidique est moindre chez les rats traités au fluconazole que chez les rats non traités. La différence la plus importante dans la quantité d'antigène est observée au jour 13 chez les rats infectés et traités au fluconazole comparés aux rats infectés et non traités (voir figure 3).

### Analyse cytologique à partir de lavements vaginaux

Les lames sont préparées sur les lavements vaginaux mis en commun et une coloration MGG est réalisée afin d'évaluer l'aspect des cellules épithéliales vaginales, la présence de bactéries et de levures et la présence de cellules pro-inflammatoires.

Au jour 2, les échantillons de contrôle sont caractérisés par la présence de cellules normales épithéliales vaginales, sans la présence de cellules inflammatoires. La principale différence chez les rats infectés par *C. albicans* est la présence d'un grand nombre de neutrophiles polynucléaires (NPN) et quelques éosinophiles indiquant l'état inflammatoire induit par l'infection à *C. albicans.*

Le traitement avec le fluconazole prévient l'infiltration inflammatoire. La présence de quelques *C. albicans* est observée correspondant probablement à la libération croissante de la levure par le traitement.

Au jour 12 après l'infection à *C. albicans,* des observations similaires sont obtenues dans le groupe de contrôle par rapport aux observations au jour J2. Dans le groupe infecté par *C. albicans,* la présence d'un grand nombre de macrophages et de quelques neutrophiles est observée, ce qui indique que *C. albicans* est bien implanté et induit l'inflammation. Chez les rats traités avec le fluconazole, aucune cellule de *C*. *albicans* n'est observée et seuls quelques macrophages et neutrophiles sont présents.

### Conclusion :

Le modèle d'infection vaginale par *C*. *albicans* et les différentes méthodes pour analyser ce modèle sont validés.

L'effet thérapeutique d'un traitement peut être analysé de façon optimale au jour J12 après l'infection à *C*. *albicans.*

### EFFICACITE DE LA LEVURE Sc I-3856

Les effets thérapeutiques préventifs et/ou curatifs de la levure Sc I-3856 sont évalués dans le modèle de la candidose vaginale développé ci-dessus et sont comparés à l'effet du fluconazole administré en mode curatif (1h, 24h et 48h après l'infection à *C*. *albicans*)*.* Pour le mode préventif, la levure Sc I-3856 est administrée 48h, 24h et 1h avant l'infection à *Candida* et une fois tous les 7 jours après l'infection à *C*. *albicans.*

Le nombre de *C*. *albicans* (en UFC) est déterminé à partir de frottis cervico-utérins et des lavements comme précédemment décrit.

Les résultats sont regroupés dans le tableau 3 ci-dessous.

| Durée en jour | 2 | 5 | 7 | 10 | 13 | 15 | 17 | 20 | 23 | 25 |
|---|---|---|---|---|---|---|---|---|---|---|
| Contrôle | 0±0 | 0±0 | 0±0 | 0±0 | 0±0 | 0±0 | 0±0 | 0±0 | 0±0 | 0±0 |
| *C*. *albicans* | 14,75±5,12 | 16,25±1,43 | 35±9,5 | 49,71±13,51 | 48,4±15,53 | 20,8±9,55 | 26±6,98 | 16±5,37 | 15,6±11,8 | 10,6±4,01 |
| *C*. *albicans* + fluco | 5,25±1,37 | 25±6,5 | 42±5,18 | 17±5,29 | 7±2,5 | 34,45±5,26 | 24,4±9,7 | 8,8±4,33 | 11,8±7,55 | 11,6±4,97 |
| *C*. *albicans* + Levure Sc I-3856 Préventif | 29,25±12,68 | 19,5±3 | 53,42±7,22 | 14,42±3,38 | 17,8±5,8 | 21,2±3,54 | 12, 2±2,08 | 15,4±5,04 | 22,8±7,41 | 18±3,76 |
| *C*. *albicans* + Levure Sc I-3856 Curatif | 18±4 | 24,75±8,375 | 36,71±10,57 | 30,14±11,20 | 33,6±13,92 | 20,8±9,55 | 13,6±7,76 | 11,6±2,72 | 9,2±2,65 | 8,4±1,36 |

Concernant l'analyse des frottis cervico-utérins et le nombre croissant de cellules de *C. albicans* sur les plaques Chrom-Agar, on observe au jour J13 une forte réduction des UFCs chez les rats infectés traités avec la levure Sc I-3856 dans un traitement préventif par rapport aux rats infectés non traités, avec respectivement 17,8 (± 5,85) vs 48,4 (± 15,53) ce qui correspond à une diminution de 63% des infections au jour J13.

Ainsi, le traitement des rats avec la levure Sc I-3856 réduit le nombre de *C*. *albicans* viables dans la muqueuse vaginale de 30% par rapport aux animaux non traités.

Le tableau 4 ci-dessous représente les valeurs ASC du jour J2 au jour J13.

| Traitement | Valeurs de l'ASC (J2 à J13) | Différence |
|---|---|---|
| *C*. *albicans* | 372,43 | |
| *C*. *albicans* + Fluconazole | 236,875 | 135,555 |
| *C. albicans* + Levure Sc I-3856 Préventif | 296,68 | 75,75 |
| *C. albicans* + Levure Sc I-3856 Curatif | 320,945 | 51,485 |

On peut conclure que les traitements préventifs avec la levure Sc I-3856 ont un effet similaire au fluconazole en réduisant respectivement de 20% et 36% les valeurs ASC par rapport aux rats non traités.

Le tableau 5 ci-dessous concerne les résultats concernant la croissance de *C. albicans* UFCs à partir de lavements.

| | | | | | | | | | | |
|---|---|---|---|---|---|---|---|---|---|---|
| Durée en jour | 2 | 5 | 7 | 10 | 13 | 15 | 17 | 20 | 23 | 25 |
| Contrôle | 0 | 0 | 0 | 0 | 0 | 0 | 0 | 0 | 0 | 0 |
| *C. albicans* | 34 | 20 | 35 | 15 | 38 | 8 | 8 | 3 | 5 | 0 |
| *C. albicans* + fluco | 9 | 53 | 17 | 3 | 1 | 6 | 7 | 3 | 1 | 4 |
| *C. albicans* +Sc-I3856 Préventif | 110 | 25 | 3 | 4 | 7 | 20 | 3 | 18 | 9 | 6 |
| *C. albicans* +ScI-3856 Curatif | 77 | 3 | 3 | 3 | 7 | 20 | 7 | 8 | 5 | 4 |

C om me dé cr it précédemment dans le modèle de candidose vaginale, les résultats peuvent être séparés en deux phases, à savoir une première étape aux jours J2-J5 correspondant à une forte libération de *C. albicans* et une seconde étape au jour J13.

L'analyse des résultats au jour J13 après l'infection à *C. albicans* montre une forte diminution du nombre des *C. albicans* viables présents dans les lavements avec le fluconazole et la levure Sc I-3856 (traitement préventif et curatif) par rapport à des rats infectés sans traitement (1 pour le fluconazole et 7 pour la levure Sc I-3856 contre 38 chez les rats infectés non traités).

L'administration de la levure Sc I-3856 (en traitement préventif ou curatif) est caractérisée par une libération rapide des *C*. *albicans* viables dans les 5 jours après l'infection suggérant que la levure Sc I-3856 peut limiter l'adhérence / invasion de *C*. *albicans* dans la muqueuse vaginale. Le taux d'élimination de *C*. *albicans* est plus rapide avec la levure Sc I-3856 qu'avec le fluconazole.

Le tableau 6 ci-dessous représente les valeurs ASC (= marqueur quantitatif de la présence de *C*. *albicans* dans la muqueuse vaginale des rats) du jour J2 au jour J13.

| Traitement | Valeurs ASC (J 2-13) |
|---|---|
| *C. albicans* | 290.5 |
| *C. albicans* + fluconazole | 199 |
| *C. albicans* + Levure Sc I-3856 Préventif | 257.6 |
| *C. albicans* + Levure Sc I-3856 Curatif | 150 |

L'analyse des valeurs ASC montre un effet plus élevé de la levure Sc I-3856 lorsqu'elle est administrée en traitement curatif après l'infection à *C. albicans,* réduisant par 2 fois la valeur d'ASC correspondant à un nombre inférieur de *C. albicans* présent dans les lavements, avec respectivement 150 contre 290,5.

La levure Sc I-3856 administrée en traitement curatif a un effet plus élevé sur l'infection à *C. albicans* que celui induit par le fluconazole, avec respectivement 150 contre 199.

Si les différentes périodes de l'expérience sont séparées respectivement aux jours J2-J5, J5-J7 et J7-J13, on observe dans la première phase du traitement (J2-J5) que l'administration de la levure Sc I-3856 en traitement préventif induit une forte libération de *C. albicans* (voir figure 4).

Il est donc fortement probable que la levure Sc I-3856, lorsqu'elle est administrée en traitement préventif, inhibe l'adhésion de *C. albicans* à la muqueuse vaginale. Ensuite, seuls quelques *C. albicans* croissent (à partir du jour J5 jusqu'au jour J13) (voir figure 4).

Le calcul du pourcentage de UFC de *C. albicans* qui croissent dans la première phase de l'expérience juste après l'infection à *C. albicans* (J2-J5), montre que 88% des UFC sont détectés en traitement préventif avec la levure Sc I-3856, 82% des UFC sont détectés en traitement curatif avec la levure Sc I-3856 et 73% avec le traitement au fluconazole.

Ces résultats indiquent que le traitement avec la levure Sc I-3856 a une efficacité plus élevée ou similaire à celle obtenue avec le fluconazole pour prévenir l'adhérence de *C*. *albicans.*

Les antigènes de *C*. *albicans* à structure mannosidique sont quantifiés dans les liquides de lavement (figure 6). Une bonne corrélation est observée entre le nombre de cellules de *C*. *albicans* (provenant des lavements vaginaux) qui croissent sur des plaques Chrom-Agar et les niveaux d'antigènes mesurés dans les mêmes lavements (figure 5).

Toutes les cellules de *C. albicans* viables et mortes sont mesurées en utilisant la même méthode d'immunodétection en contraste avec la méthode conventionnelle de milieu sélectif permettant la multiplication des cellules viables de *C. albicans.*

Pour l'étude cytologique des lavements vaginaux, des lames sont préparées à partir des lavements vaginaux mis en commun et une coloration MGG est réalisée.

L'aspect des cellules vaginales épithéliales, la présence de bactéries et de levures et la présence de cellules pro-inflammatoires sont évalués par observation (résultats non montrés).

Au jour J2, le groupe de contrôle est caractérisé par la présence de cellules vaginales épithéliales normales, sans la présence de cellules inflammatoires. La principale différence chez les rats infectés par *C. albicans* est la présence d'un nombre élevé de neutrophiles polynucléaires (NPN) et quelques éosinophiles indiquant l'état inflammatoire induit par l'infection à *C. albicans.*

Le traitement avec le fluconazole empêche l'infiltration inflammatoire. La présence de quelques *C. albicans* est observée correspondant probablement à la libération augmentée de *C. albicans* par le traitement.

Au jour J12 après l'infection par *C. albicans,* des observations similaires sont obtenues dans le groupe contrôle par rapport au jour J2. Chez les rats infectés à *C. albicans,* la présence d'un grand nombre de macrophages et de quelques neutrophiles est observée, indiquant que les *C. albicans* sont bien implantés et induisent l'inflammation. Chez les rats traités avec le fluconazole, plus aucun *C. albicans* n'est observé et quelques macrophages et neutrophiles sont observés. Chez les rats traités avec le fluconazole, la présence de *C.albicans* est observée, mais aucune cellule immunitaire n'est trouvée (résultats non montrés).

Chez les rats traités de façon préventive avec la levure Sc I-3856, des macrophages, des neutrophiles et des éosinophiles sont observés deux jours après l'infection par *C*. *albicans,* mais à une densité moindre que celle observée chez les rats infectés par *C*. *albicans* (résultats non montrés).

Chez les rats traités de façon curative avec la levure Sc I-3856, aucun macrophage et aucun éosinophile n'est observé, mais un nombre élevé de neutrophiles est présent, ainsi que cela a été observé chez les rats infectés par *C*. *albicans.*

La présence de *C*. *albicans* est observée chez les rats infectés par *C*. *albicans* et traités à l'aide du fluconazole ou à l'aide de la levure Sc I-3856 (en traitement préventif ou curatif), mais n'est pas observée chez les rats infectés par *C. albicans* et non traités, ce qui démontre l'efficacité du traitement induisant le relarguage ou la prévention de l'adhésion de *C. albicans* à la muqueuse vaginale.

Au jour J12 après l'infection à *C. albicans,* des observations similaires sont obtenues dans le groupe de contrôle par rapport aux observations au jour J2. Chez les rats infectés à *C. albicans,* la présence d'un grand nombre de macrophages et de quelques neutrophiles est observée indiquant que les *C. albicans* sont bien implantés et induisent une inflammation. Chez les rats traités avec le fluconazole, plus aucun *C. albicans* n'est observé et quelques macrophages et neutrophiles sont observés.

Chez les rats traités de façon préventive avec la levure Sc I-3856, aucun macrophage n'est observé mais la présence de neutrophiles est observée.

Chez les rats traités de façon curative avec la levure Sc I-3856, la présence de macrophages et de neutrophiles est observée. Cependant, comparé aux rats infectés par *C*. *albicans,* moins de macrophages sont observés, ce qui indique une diminution de l'inflammation due à l'administration de la levure Sc I-3856 (résultats non montrés).

### CONCLUSION

La Demanderesse a développé un nouveau modèle de candidose vaginale chez la femelle du rat. L'intensité maximale de l'infection vaginale est observée 12 à 13 jours après l'administration de *Candida albicans.* Le contrôle positif à l'aide du traitement antimycotique au fluconazole permet de prévenir complètement l'infection par *Candida albicans 12* jours après ladite administration et augmente la libération de *Candida albicans* dans la lumière vaginale particulièrement durant les six premiers jours de l'infection.

Le traitement préventif à l'aide de la levure Sc I-3856 montre également un fort effet thérapeutique permettant de réduire l'infection vaginale 12 à 13 jours après l'administration de *Candida albicans* chez plus de 60% des rats traités en comparaison des rats non traités. Cet effet préventif est très probablement dû à une libération rapide de *Candida albicans* dans la lumière vaginale, qui a lieu durant les 4 premiers jours qui suivent l'infection à l'aide de *Candida albicans.*

Le traitement curatif est également efficace mais cependant avec une efficacité légèrement moindre par rapport à celle du traitement préventif.

Comparé au traitement à l'aide du fluconazole, le traitement préventif à l'aide de la levure Sc I-3856 conduit à une libération plus rapide de *Candida albicans* dans la lumière vaginale des rats présentant une candidose vaginale.

La diminution des *Candida albicans* viables dans la muqueuse vaginale des rats après 12 à 13 jours est importante dans les deux groupes de rats traités respectivement avec du fluconazole ou avec la levure Sc I-3856, bien que légèrement supérieure à l'aide du traitement au fluconazole. Cette diminution légèrement supérieure est probablement due à l'effet antimycotique de la molécule fluconazole.

## Revendications

1. Souche *Saccharomyces cerevisiae* déposée auprès de la CNCM sous le numéro I-3856, pour son utilisation dans la prévention et/ou le traitement des mycoses vaginales.

2. Levure obtenue par culture de la souche *Saccharomyces cerevisiae* déposée auprès de la CNCM sous le numéro I-3856, pour son utilisation dans la prévention et/ou le traitement des mycoses vaginales.

3. Levure pour une utilisation selon la revendication 2, ladite levure se présentant sous forme de levure fraîche ou sous forme de levure sèche.

4. Levure pour une utilisation selon la revendication 3, ladite levure se présentant sous forme de levure sèche active ou de levure sèche instantanée.

5. Levure pour une utilisation selon la revendication 2, ladite levure se présentant sous forme de levure morte.

6. Dérivé de la levure obtenue par culture de la souche *Saccharomyces cerevisiae* déposée auprès de la CNCM sous le numéro I-3856, pour son utilisation dans la prévention et/ou le traitement des mycoses vaginales, ledit dérivé étant choisi parmi la paroi de ladite levure, les glucanes pariétaux de ladite levure, les mannoprotéines pariétales de ladite levure ou leurs mélanges.

7. Souche, levure ou dérivé pour une utilisation selon l'une des revendications 1 à 6, dans laquelle la mycose vaginale est une candidose vaginale.

8. Souche, levure ou dérivé pour une utilisation selon la revendication 7 dans laquelle la candidose vaginale est due à des champignons choisis dans le groupe comprenant *Candida albicans, Candida tropicalis, Candida pseudotropicalis, Candida krusei, Candida glabrata* et leurs mélanges.

9. Composition comprenant une levure obtenue par culture de la souche *Saccharomyces cerevisiae* déposée à la CNCM sous le numéro I-3856 et/ou un dérivé de ladite levure, ledit dérivé étant choisi parmi la paroi de ladite levure, les glucanes pariétaux de ladite levure, les mannoprotéines pariétales de ladite levure ou leurs mélanges, et éventuellement au moins un excipient pharmaceutiquement acceptable, pour une utilisation dans la prévention et/ou le traitement de mycoses vaginales.

10. Composition pour une utilisation selon la revendication 9, qui est destinée à une administration par voie orale ou par voie vaginale.

11. Composition pour une utilisation selon la revendication 9 ou 10, dans laquelle ladite levure est sous forme de levure morte et est administrée quotidiennement dans une quantité de 1 mg à 10 g, et de préférence de 100 mg à 1 g.

12. Composition pour une utilisation selon la revendication 9 ou 10, dans laquelle ladite levure est sous forme de levure sèche ou de levure fraîche, et est administrée quotidiennement dans une quantité de 1.10⁷ à 1.10¹¹ UFC, et de préférence de 1.10⁹ à 1.10¹⁰ UFC.

13. Composition pour une utilisation selon l'une quelconque des revendications 9 à 12, comprenant ladite levure et/ou ledit dérivé en association avec un autre principe actif.

14. Composition pour une utilisation selon la revendication 13, dans laquelle l'autre principe actif est choisi dans le groupe des principes actifs ayant une activité apaisante, anti-irritante, analgésique, antalgique, anti-inflammatoire, cicatrisante, antifongique, antimycosique et/ou antimycotique.

## Patentansprüche

1. *Saccharomyces cerevisiae-Stamm,* der bei der CNCM unter der Nummer I-3856 hinterlegt ist, zur Verwendung bei der Prävention und/oder Behandlung von Vaginalmykosen.

2. Hefe, welche durch Kultivierung des *Saccharomyces cerevisiae*-Stammes, der bei der CNCM unter der Nummer I-3856 hinterlegt ist, erhalten wurde, zur Verwendung bei der Prävention und/oder Behandlung von Vaginalmykosen.

3. Hefe zur Verwendung nach Anspruch 2, wobei die Hefe in Form von Frischhefe oder Trockenhefe vorliegt.

4. Hefe zur Verwendung nach Anspruch 3, wobei die Hefe in Form von aktiver Trockenhefe oder Instant-Trockenhefe vorliegt.

5. Hefe zur Verwendung nach Anspruch 2, wobei die Hefe in Form von abgetöteter Hefe vorliegt.

6. Hefederivat, welches durch Kultivierung des *Saccharomyces cerevisiae*-Stammes, der bei der CNCM unter der Nummer I-3856 hinterlegt ist, erhalten wurde, zur Verwendung bei der Prävention und/oder Behandlung von Vaginalmykosen, wobei das Derivat gewählt ist aus der Hefezellwand, den parietalen Glucanen der Hefe, den parietalen Mannoproteinen der Hefe oder deren Mischungen.

7. Stamm, Hefe oder Derivat zur Verwendung nach einem der Ansprüche 1 bis 6, wobei die Vaginalmykose eine Vaginalcandidose ist.

8. Stamm, Hefe oder Derivat zur Verwendung nach Anspruch 7, wobei die Vaginalcandidose auf Pilze zurückzuführen ist, gewählt aus der Gruppe umfassend *Candida albicans, Candida tropicalis, Candida pseudotropicalis, Candida krusei, Candida glabrata* und deren Mischungen.

9. Zusammensetzung, umfassend eine Hefe, die durch Kultivierung des *Saccharomyces cerevisiae-Stammes,* der bei der CNCM unter der Nummer I-3856 hinterlegt ist, und/oder eines Derivats der Hefe erhalten wird, wobei das Derivat aus der Hefezellwand, den parietalen Glucanen der Hefe, den parietalen Mannoproteinen der Hefe oder deren Mischungen und gegebenenfalls mindestens einem pharmazeutisch akzeptablen Hilfsstoff zur Verwendung bei der Prävention und/oder Behandlung von Vaginalmykosen gewählt ist.

10. Zusammensetzung zur Verwendung nach Anspruch 9, die zur oralen oder vaginalen Verabreichung bestimmt ist.

11. Zusammensetzung zur Verwendung nach Anspruch 9 oder 10, wobei die Hefe in Form von abgetöteter Hefe vorliegt und täglich in einer Menge von 1 mg bis 10 g und vorzugsweise von 100 mg bis 1 g verabreicht wird.

12. Zusammensetzung zur Verwendung nach Anspruch 9 oder 10, wobei die Hefe in Form von Trocken- oder Frischhefe vorliegt und täglich in einer Menge von 1.10⁷ bis 1.10¹¹ KBE, vorzugsweise 1.10⁹ bis 1.10¹⁰ KBE, verabreicht wird.

13. Zusammensetzung zur Verwendung nach einem der Ansprüche 9 bis 12, umfassend die Hefe und/oder das Derivat in Kombination mit einem anderen Wirkstoff.

14. Zusammensetzung zur Verwendung nach Anspruch 13, wobei der andere Wirkstoff aus der Gruppe der Wirkstoffe mit einer beruhigenden, reizhemmenden, analgetischen, schmerzstillenden, entzündungshemmenden, heilenden, antifungalen und/oder antimykotischen Wirkung gewählt ist.

## Claims

1. The *Saccharomyces cerevisiae* strain deposited at the CNCM under number 1-3856, for use thereof in the prevention and/or treatment of vaginal mycoses.

2. A yeast obtained by culturing the *Saccharomyces cerevisiae* strain deposited at the CNCM under number 1-3856, for use thereof in the prevention and/or treatment of vaginal mycoses.

3. The yeast for its use as claimed in claim 2, which is in the form of fresh yeast or in the form of dry yeast.

4. The yeast for its use as claimed in claim 3, which is in the form of active dry yeast or instant dry yeast.

5. The yeast for its use as claimed in claim 2, which is in the form of dead yeast.

6. A derivative of the yeast obtained by culturing the *Saccharomyces cerevisiae* strain deposited at the CNCM under number 1-3856, for use thereof in the prevention and/or treatment of vaginal mycoses, said derivative being selected from the cell wall of said yeast, the cell wall glucans of said yeast, the cell wall mannoproteins of said yeast or mixtures thereof.

7. The strain, yeast or derivative for its use as claimed in one of claims 1 to 6, wherein the vaginal mycoses is a vaginal candidoses.

8. The strain, yeast or derivative for use as claimed in claim 7, wherein the vaginal candidoses is due to fungi selected from the group comprising *Candida albicans, Candida tropicalis, Candida pseudotropicalis, Candida krusei, Candida glabrata* and mixtures thereof.

9. A composition comprising a yeast obtained by culturing the *Saccharomyces cerevisiae* strain deposited at the CNCM under number 1-3856 and/or a derivative of said yeast, said derivative being selected from the cell wall of said yeast, the cell wall glucans of said yeast, the cell wall mannoproteins of said yeast or mixtures thereof, and optionally at least one pharmaceutically acceptable excipient, for use in the prevention and/or treatment of vaginal mycoses.

10. The composition for its use as claimed in claim 9, which is intended for administration by the oral route or by the vaginal route.

11. The composition for its use as claimed in claim 9 or 10, wherein said yeast is in the form of dead yeast, for daily use in an amount from 1 mg to 10 g, and preferably from 100 mg to 1 g.

12. The composition for its use as claimed in claim 9 or 10, wherein said yeast is in the form of dry yeast or fresh yeast, for daily use in an amount from 1.10⁷ to 1.10¹¹ CFU, and preferably from 1.10⁹ to 1.10¹⁰ CFU.

13. The composition for its use as claimed in any one of claims 9 to 12, comprising said yeast and/or said derivative in combination with another active principle.

14. The composition for its use as claimed in claim 13, wherein the other active principle is selected from the group of active principles having a soothing, anti-irritant, analgesic, anti-inflammatory, wound-healing, antifungal, and/or antimycotic activity.
